# EUROPÄISCHE PATENTSCHRIFT

(11) **EP 1 937 285 B1**
(45) Veröffentlichungstag und Bekanntmachung des Hinweises auf die Patenterteilung: **06.04.2011**
(21) Anmeldenummer: 06805400.6
(22) Anmeldetag: 09.10.2006
(51) Int. Cl.: A61K 33/06, A61K 36/185

(54) **MITTEL ZUR THERAPIE UND PROPHYLAXE DES DIABETES MELLITUS**
AGENT FOR THE THERAPY AND PROPHYLAXIS OF DIABETES MELLITUS
AGENT POUR LA THERAPIE ET LA PROPHYLAXIE DU DIABETE SUCRE

(30) Priorität: 07.10.2005 DE 102005048483
(43) Veröffentlichungstag der Anmeldung: 02.07.2008
(73) Patentinhaber: Schulz, Jörg, 13127 Berlin (DE); Bendzko, Peter, Dr., 12623 Berlin (DE)
(72) Erfinder: BENDZKO, Peter, 12623 Berlin (DE); SCHULZ, Jörg, 13127 Berlin (DE); GULBIN, Klaus, 19089 Crivitz (DE)
(74) Vertreter: Baumbach, Friedrich
(86) Internationale Anmeldenummer: PCT/DE2006/001782
(87) Internationale Veröffentlichungsnummer: WO 2007/042006

(56) Entgegenhaltungen:
- WO-A-2006/122541
- WO-A1-01/95920
- SVERKO VISNJA ET AL: "NATURAL MICRONISED CLINOPTILOLITE AND CLINOPTILOLITE MIXTURES WITH URTICA DIOICA L. EXTRACT AS POSSIBLE ANTIOXIDANTS" FOOD TECHNOLOGY AND BIOTECHNOLOGY, ZAGREB, HR, Bd. 42, Nr. 3, Juli 2004 (2004-07), Seiten 189-192, XP009072328 ISSN: 1330-9862
- ANONYM: "Megamin - was ist das? -Teil 2-" INTERNET ARTIKEL, [Online] XP002415756 Gefunden im Internet: URL:http://www.megamin-bestellen.de/Antiox idans.Zeolith-Klinoptilolith.Mehr-ueber-Me gamin.html> [gefunden am 2007-01-19]
- FAULHABER MARTIN: 'Neue Aspekte des Höhentrainings' INTERNET ARTIKEL, [Online] 30 März 2005, Gefunden im Internet: <URL:http://www.tibs.at/spn/pdf/Hoehentrain ing.pdf> [gefunden am 2009-09-17]
- FÜESSL H.S.: 'Abnehmen bei Diabetes Typ 2: Viel wichtiger als Blutzucker-Kosmetik = Weight reduction in type 2 diabetes. Much more important than blood glucose monitoring' MMW FORTSCHRITT DER MEDIZIN Bd. 144, Nr. 17, 25 April 2002, Seiten 4 - 8, XP009122888

## Beschreibung

Die Erfindung betrifft ein Mittel zur Therapie (und Prophylaxe) des Diabetes mellitus. Anwendungsgebiete der Erfindung sind die Medizin und die pharmazeutische Industrie.

### Einleitung

Diabetes mellitus wird durch absoluten oder relativen Mangel an Insulin hervorgerufen. Er führt u.a. zur Zunahme der Plasmaglucosekonzentration. Je nach Ursache und Verlauf unterscheidet man mehrere Typen des Diabetes mellitus.

Bei Typ-I-Diabetes (insulinabhängiger Diabetes mellitus) liegt absoluter Insulin-Mangel vor. Ursache ist eine Läsion der B-Zellen in den Pankreasinseln, meist durch eine Autoimmunerkrankung hervorgerufen, die durch eine Virusinfektion ausgelöst werden kann.

Tierexperimentelle Untersuchungen weisen darauf hin, dass freie Radikale zur Läsion der B-Zellen führen. Bei Entzündung der Pankreasinseln werden freie Sauerstoff-Radikale in toxischen Mengen von den infiltrierten Makrophagen und Endothelzellen freigesetzt. Die B-Zellen des Pankreas werden durch freie Sauerstoff-Radikale zerstört, denn sie besitzen nur ungenügend Abwehr gegenüber freien Radikalen.

Der Typ-II-Diabetes (insulinunabhängiger Diabetes mellitus) ist die weitaus häufigste Form des Diabetes. In Deutschland leben zur Zeit ca. 200.000 Typ-I-Diabetiker, dagegen ca. 4 Millionen Typ-II-Diabetiker (MICHAELIS, 1985). Hier liegt ein relativer Mangel an Insulin vor. Die Insulinausschüttung kann normal oder gesteigert sein, doch immer zeigen die Zielorgane gegenüber Insulin verminderte Empfindlichkeit. Patienten mit Typ-II-Diabetes sind meist übergewichtig. Adipositas ist Folge genetischer Disposition, zu reichlicher Nahrungszufuhr und zu geringer Bewegung.

Deutlich häufiger als bei Nichtdiabetikern bestehen bei Typ-II-Diabetikern Hypertonie und Fettstoffwechselstörungen (bezeichnet als Metabolisches Syndrom, REAVEN, 1991). Bisher betraf der Typ-II-Diabetes vorwiegend ältere Menschen ("Altersdiabetes") - seit einigen Jahren gibt es eine alarmierende Inzidenzzunahme auch bei jungen Menschen der Allgemeinbevölkerung (Jahrestagung der Amerikanischen Diabetes Gesellschaft, San Diego, 1999).

Auf Grund dieser Stoffwechselentgleisungen kommt es dann schließlich zu Makro- und Mikrozirkulationsstörungen mit den typischen Organmanifestationen. Die Folgen sind dann insbesondere Augenschädigungen, Nierenerkrankungen, ischämische Erkrankungen am Herzen und peripherem Gefäßsystem sowie Auftreten cerebraler und neurologischer Schädigungen.

Untersuchungen an Tieren lassen vermuten, dass freie Radikale in der Pathogenese des Diabetes mellitus die Destruktion der Zellinseln im Pankreas fördern. Reaktive Sauerstoffzwischenprodukte, in toxischen Mengen von Endothelzellen und eingewanderten Makrophagen im Laufe der Entzündung des Betazellgewebes im Pankreas freigesetzt, führen zu massiver Schädigung und Vernichtung von Betazellen. Zahlreiche Untersuchungen lassen vermuten, dass der diabetische Zustand mit oxidativem Stress verbunden ist.

Oxidativer Stress ist auch ein Synonym für Diabetes. Er ist dabei für folgende Komplikationen verantwortlich: Polyneuropathie, Retinopathie und Angiopathie. Zwar wurden einige Hundert Arbeiten über die Bedeutung von freien Radikalen und Anwendung von Antioxidanzien bei der Diabetes veröffentlicht. Antioxidanzien sind jedoch noch immer nicht in die offiziellen Protokolle der Antidiabetestherapie aufgenommen.

Viele Entwicklungsforschungen an Versuchstieren haben die Schutzwirkung von Komponenten bestätigt, die Radikale in den Zellinseln entfernen (z.B. Nikotinamid). Marker für oxidativen Stress zeigen sich bei Zuckererkrankung gerade dort, wo die Menge an Vitamin C reduziert ist.

Zum Schutz gegen freie Radikale verfügt der Organismus über ein antioxidatives Schutzsystem, er entwickelte die Antioxidanzien. Die wichtigsten Antioxidanzien sind die antioxidativen Enzyme Glutathionperoxidase und Superoxiddismutase. Sie enthalten als wesentliche Bestandteile Selen, Zink, Kupfer und Mangan sowie nichtenzymatische Antioxidanzien wie Alpha-Tocopherol (Vitamin E), Beta-Carotin (Provitamin A), Ascorbinsäure (Vitamin C), Melatonin und Glutathion und andere mehr.

Antioxidative Schutzstoffe beeinflussen sich dabei wechselseitig. Beispielsweise wird Vitamin E (im Blut als D-Alpha-Tocopherol vorkommend) durch Vitamin C (Ascorbinsäure) "regeneriert".

Zellmembranständiges fettlösliches Vitamin E wird als erste Abwehrlinie gegen die Peroxidation angesehen. Als Radikalfänger beendet es Kettenreaktionen in der Zellmembran und begrenzt damit Zellmembranschäden auf umschriebene Gebiete.

Das wasserlösliche Vitamin C (Ascorbinsäure) schützt nicht nur die Zellmembran durch Vitamin E-Regeneration, sondern auch das Zellinnere, insbesondere den Zellkern, indem es im Zytoplasma der Zelle als Redoxkatalysator wirkt und freie Radikale "neutralisiert". Dabei geht die Ascorbinsäure durch Abgabe von Wasserstoff in ihr Oxydationsprodukt über, die Dehydroascorbinsäure. Diese verändert die Zellstruktur und kann zu neurotoxischen Wirkungen führen. In der normalen Zelle ist diese Reaktion reversibel.

Der geschädigte Stoffwechsel der Askorbinsäure (Vitamin C) ist ebenfalls mit der Zuckerkrankheit verbunden. Askorbat wird in vivo für die Regeneration von Vitamin E benötigt und kann zu Dehydroaskorbinsäure oxidiert werden, was die Zellstruktur verändern und neurotoxisch wirken kann. Im gesunden Gewebe wandelt sich die Dehydroaskorbinsäure wieder in Askorbinsäure um. Die erhöhte Entstehung von großen Mengen Dehydroaskorbinsäure und geringer Mengen Askorbinsäure führt zur erhöhten Empfindlichkeit der Zellen gegenüber Oxidationsschäden. Andere Untersuchungen zeigen, dass ein hohes Niveau der Glucose bei der Zuckerkrankheit mit dem Einbringen von Askorbinsäure einher geht, was ein niedriges Niveau von Askorbat in den Zellen zur Folge hat.

Die Hauptfrage, die bei evident erhöhtem Oxidationsstress zu stellen ist, ist die, ob die verschiedenen Indexe, welche auf das Wirken freier Radikale hindeuten, ein Zeichen von Oxidationsstress als ätiologischer Faktor sind oder wurden sie sekundär durch das geschädigte Gewebe erzeugt. Außerdem kann die Evidenz von primärem Oxidationsstress aus der Untersuchung antioxidanter Reserven und aus der Statusverbesserung bei der Zugabe von Antioxidanten herrühren. In Verbindung damit muss gesagt werden, dass das Niveau der Askorbinsäure im Plasma gesichert bei Diabetikern gemindert ist und das sowohl bei Menschen- so auch bei Tiergruppen, wogegen das Dehydroaskorbat, primär ein Oxidationsprodukt, erhöht ist. Dagegen ist das Niveau der Askorbinsäure in den einfachnuklearen Leukozyten von Patienten mit Diabetes verringert.

Die Lebenserwartung eines 40-jährigen mit neu diagnostiziertem Diabetes ist durchschnittlich um 8 Jahre reduziert. 75% aller Typ 2-Diabetiker (bzw. 35% aller Typ 1-Diabetiker) sterben an kardiovaskulären Komplikationen. Im Vergleich zu Personen ohne Diabeteserkrankung ist bei Typ 2-Diabetikern das Risiko für eine KHK (Koronare HerzKrankheit) um mehr als das 3-fache und für eine zerebrovaskuläre Erkrankung um mehr als das Doppelte erhöht.

Zusammenfassend kann festgestellt werden, dass es noch keine befriedigende Behandlungsmethode für Diabeteserkrankungen gibt und damit ein gesundheitspolitisches Problem ersten Ranges noch auf seine Lösung wartet.

Die Erfindung hat das Ziel, ein neuartiges Mittel zur Therapie und Prophylaxe von Diabetes mellitus zu entwickeln.

Dieses Ziel wird durch das im Anspruch 1 beschriebene Mittel erreicht. Überraschenderweise wurde gefunden, dass ein Mittel, umfassend physikalisch und/oder chemisch aktivierte natürliche oder synthetische Zeolithe, Extrakte von Pflanzen der Gattung Urticaceae, Omega-3-Fettsäuren und gegebenenfalls weitere Stoffe unter den Bedingungen normobarer Hypoxie, für die Therapie und Prophylaxe von Diabetes mellitus geeignet ist. Das Mittel ist sowohl für Typ I wie für Typ II-Diabetes anwendbar.

Erfindungsgemäß werden sowohl natürliche als auch synthetische Zeolithe verwendet, bevorzugt eine oder mehrere der folgenden Zeolitharten: Heulandit/Klinoptliolith, Natrolith oder Thomsonit. Diese Zeolitharten enthalten ca. 20-30% Magnesium und Calziumanteil. Besonders bevorzugt ist mikronisiertes Heulandit/ Klinoptilolith mit einem Teilchendurchmesser von weniger als 5 µm.

Die Bestandteile des erfindungsgemäßen Mittels sind in folgenden Mengen enthalten: Mineral 70-98% und Extrakte von Pflanzen der Gattung Urticaceae 2-30%, bevorzugt sind Zeolith 75% und Brennnesselextrakt 25%.

Die Verwendung des Mittels erfolgt in der Regel in Form eines Nahrungsergänzungsmittels, gegebenenfalls in Kombination mit weiteren Stoffen. Bevorzugt ist die orale Einnahme in Form von Pulver, Tabletten oder Kapseln.

Mit dem erfindungsgemäßen Mittel wurden folgende klinischen Resultate erreicht

Aktivierter Klinoptilolith in Verbindung mit Extrakten von Pflanzen der Gattung Urticaceae entfaltet eine starke antioxidative Wirkung, ca. 20-fach stärkerals Vitamin C oder Vitamine E. Daher führt die Einnahme von aktiviertem Klinoptilolith in Verbindung mit Extrakten von Pflanzen der Gattung Urticaceae zu einer enorm erhöhten antioxidativen Kapazität des Organismus.

Bei den Patienten mit Diabetes mellitus, die aktivierten Klinoptilolith mit Extrakten von Pflanzen der Gattung Urticaceae einnahmen, wurden die Aktivitäten der endogenen antioxidativen Enzyme SOD, Glutathionperoxidase (GPx) und Glutathionreduktase (GR) mit Hilfe des kommerziell erhältlichen Reagens ABTS der Firma Randox bestimmt.

Bei gesunden Personen, die täglich 4 Kapseln aktivierten Klinoptilolith mit Brennesselextrakt einnahmen, lag der mittlere TAS-Wert mit 1,55 mmol/l eindeutig höher im Vergleich zu den Personen ohne aktivierte Klinoptilolith und Brennesselextrakt-Einnahme.

Alle bisherigen Untersuchungen deuten darauf hin, dass Hyperglykämie oxidativen Stress mit Schwächung des antioxidativen Schutzsystems bewirkt. Folgen sind Schäden an den peripheren Neuronen und Gefäßen, die zur Entwicklung von Spätkomplikationen des Diabetes mellitus führen wie Polyneuropathie, Mikroangiopathie, insbesondere Retinopathie und Glomerulosklerose. Durch Erhöhung des endogenen antioxidativen Potenzials durch Einnahme exogener Antioxidanzien kann diesen Komplikationen in starkem Maß vorgebeugt und so der negative Effekt des oxidativen Stresses reduziert werden.

Auffallend ist, dass vorrangig Patienten mit insulinunabhängigem Diabetes auf die Einnahme von antioxidativ wirkendem aktivierten Klinoptilolith mit Brennesselextrakt ansprachen.

Ferner betrifft die Erfindung die Erkenntnis, dass Diabetiker vom Typ 1 und Typ 2 davon profitieren, dass die Zelle in eine relative Sauerstoffschuld gebracht wird. Das kann durch ein körperliches Bewegungsprogramm in der Höhe, in Höhentrainingszentren unter normobarer Hypoxie oder mit einem veränderten respiratorischen Quotienten (RQ kleiner oder gleich 0,85) durch bestimmte Luftveränderungen bei der Einatmung (geänderte Sauerstoff- und Stickstoffkonzentration) erreicht werden. Durch Gabe von aktivierten Zeolithen auch in Kombination mit Extrakten von Pflanzen der Gattung Urticaceae und Omega-3-Fettsäuren ist eine erhebliche Verbesserung des Gesamtbildes des Metabolischen Syndroms und insbesondere des Diabetes mellitus erreichbar. Erwartungsgemäß lässt sich durch den Einsatz der vorliegenden Erfindung insgesamt der Gesundheitszustand von Diabetikern erheblich verbessern.

Die Erfindung soll im Folgenden durch Anwendungsbeispiele veranschaulicht werden.

### Anwendungsbeispiel 1

### Klinische Fallkontrollstudie mit aktiviertem Klinoptilolith mit Brennesselextrakt bei Patienten mit Diabetes mellitus Typ 2

Auf Grund der positiven Untersuchungsergebnisse sowohl im tierexperirnentellen als auch im klinischen Bereich, erfolgte eine klinische Fallkontrollstudie an 30 Patienten mit gesichertem, bereits jahrelang bestehendem Diabetes mellitus Typ 2 unter ambulanten Behandlungs- und Kontrollbedingungen.

### Methodisches Vorgehen

30 Patienten mit Diabetes mellitus Typ 2 erhielten 3 Monate lang 3 x 3 Kapseln aktivierten Klinoptilolith mit Brennesselextrakt täglich. Kontrolluntersuchungen erfolgten zu Beginn der Fallbeobachtungsstudie und nach 6 und 12 Wochen.

Laborchemisch wurden untersucht:
- C-Peptid
- Insulin
- Proinsulin
- HbA₁c
- Cholesterin
- HDL-Cholesterin
- LDL-Cholesterin
- Triglyceride
- Blutzucker

Alle Untersuchungen (Einschluss 1. und 2. Kontrolle) erfolgten jeweils innerhalb von 2 Konsultationen in einer allgemeinmedizinischen Praxis und wurden ärztlich überwacht.

### Ergebnisse

Bei 30 Patienten mit jahrelang bestehendem Diabetes mellitus Typ 2 wurde nach 3-monatiger Behandlung mit aktiviertem Klinoptilolith mit Brennesselextrakt festgestellt, dass sich das C-Peptid, HbA₁ und die Triglyceride signifikant positiv entwickelt hatten. Diese Ergebnisse lassen Rückschlüsse auf neue Therapievarianten beim Diabetes mellitus zu. Bei stark übergewichtigen Patienten (BROCA-Index > 18) war die Wirksamkeit von 3 x 3 Kapseln aktiviertem Klinoptilolith mit Brennesselextrakt weniger gut nachweisbar, hier ist die Dosierung in Abhängigkeit vom Körpergewicht zu variieren.

Die wichtigsten Daten sind den nachfolgenden Tabellen zu entnehmen.

**Tabelle 1: Mittelwerte (MW) und Standardabweichung (SD) der Laborwerte nach Diabetes-Typ zu den einzelnen Untersuchungszeitpunkten**

| Laborwerte | Diabetes Typ | Anzahl | 1.Untersuchung | | 2.Untersuchung | | 3.Untersuchung | |
|---|---|---|---|---|---|---|---|---|
| | | | MW | SD | MW | SD | MW | SD |
| C-Peptid | diätisch | 8 | 3,89 | 2,65 | 4,54 | 2,57 | 5,13 | 1,92 |
| | Tabletten | 12 | 3,77 | 1,79 | 3,96 | 2,14 | 5,43 | 2,39 |
| | Insulin | 10 | 2,51 | 1,83 | 3,11 | 2,02 | 3,00 | 1,06 |
| | Gesamt | 30 | 3,38 | 2,08 | 3,83 | 2,22 | 4,54 | 2,15 |
| Insulin | diätisch | 8 | 17,31 | 10,92 | 33,28 | 32,95 | 22,90 | 13,17 |
| | Tabletten | 12 | 21,08 | 16,73 | 29,49 | 27,97 | 30,75 | 27,69 |
| | Insulin | 10 | 41,63 | 23,55 | 74,58 | 46,48 | 51,65 | 40,38 |
| | Gesamt | 30 | 26,92 | 20,53 | 45,53 | 40,84 | 35,62 | 31,33 |
| Proinsulin | diätisch | 8 | 39,58 | 23,14 | 32,90 | 21,84 | 34,71 | 25,53 |
| | Tabletten | 12 | 24,08 | 19,69 | 25,88 | 20,84 | 23,13 | 20,48 |
| | Insulin | 10 | 22,04 | 14,07 | 18,14 | 14,02 | 14,58 | 8,96 |
| | Gesamt | 30 | 27,52 | 19,82 | 25,17 | 19,35 | 23,37 | 20,09 |
| HbA1C | diätisch | 8 | 6,61 | 0,65 | 6,65 | 0,80 | 6,85 | 0,92 |
| | Tabletten | 12 | 7,13 | 1,20 | 7,05 | 1,21 | 7,11 | 0,93 |
| | Insulin | 10 | 6,85 | 0,60 | 6,63 | 0,70 | 6,86 | 0,67 |
| | Gesamt | 30 | 6,90 | 0,90 | 6,80 | 0,95 | 6,96 | 0,83 |
| Cholesterin | diätisch | 8 | 6,34 | 1,59 | 6,26 | 1,31 | 6,08 | 0,91 |
| | Tabletten | 12 | 5,60 | 1,38 | 5,95 | 1,51 | 5,73 | 1,61 |
| | Insulin | 10 | 4,93 | 0,70 | 5,02 | 0,72 | 4,83 | 0,66 |
| | Gesamt | 30 | 5,57 | 1,34 | 5,72 | 1,31 | 5,52 | 1,26 |
| HDL-Cholesterin | diätisch | 8 | 1,33 | 0,32 | 1,46 | 0,32 | 1,33 | 0,32 |
| | Tabletten | 12 | 1,31 | 0,29 | 1,37 | 0,31 | 1,36 | 0,33 |
| | Insulin | 10 | 1,21 | 0,24 | 1,19 | 0,23 | 1,22 | 0,25 |
| | Gesamt | 30 | 1,28 | 0,28 | 1,33 | 0,30 | 1,30 | 0,30 |
| LDL-Cholesterin | diätisch | 7 | 3,93 | 1,35 | 3,72 | 1,19 | 3,36 | 0,70 |
| | Tabletten | 11 | 3,59 | 1,16 | 3,73 | 1,18 | 3,47 | 1,23 |
| | Insulin | 9 | 3,11 | 0,69 | 3,10 | 0,69 | 2,94 | 0,69 |
| | Gesamt | 27 | 3,52 | 1,09 | 3,50 | 1,04 | 3,26 | 0,97 |
| Triglyceride | diätisch | 8 | 2,74 | 1,58 | 3,05 | 1,81 | 3,49 | 2,46 |
| | Tabletten | 12 | 1,79 | 1,34 | 1,86 | 0,82 | 2,28 | 1,48 |
| | Insulin | 10 | 1,65 | 1,60 | 1,60 | 0,78 | 1,75 | 1,21 |
| | Gesamt | 30 | 1,99 | 1,51 | 2,09 | 1,26 | 2,42 | 1,80 |
| Blutzucker | diätisch | 8 | 7,32 | 2,18 | 8,44 | 2,39 | 8,61 | 2,22 |
| | Tabletten | 12 | 10,16 | 4,64 | 9,91 | 3,45 | 11,25 | 3,97 |
| | Insulin | 10 | 9,13 | 4,78 | 9,50 | 4,53 | 9,59 | 4,19 |
| | Gesamt | 30 | 9,06 | 4,21 | 9,38 | 3,55 | 9,99 | 3,72 |

**Tabelle 2: Mittelwerte (MW) und Standardabweichung (SD) der Laborwerte nach Broca-Index-Gruppen zu den einzelnen Untersuchungszeitpunkten. Dabei bedeutet "leicht erhöht" bis 18 kg Übergewicht und "erhöht" 18 kg und mehr Übergewicht.**

| Laborwerte | Broca-Index | Anzahl | 1.Untersuchung | | 2.Untersuchung | | 3.Untersuchung | |
|---|---|---|---|---|---|---|---|---|
| | | | MW | SD | MW | SD | MW | SD |
| C-Peptid | normal | 8 | 3,10 | 1,74 | 3,20 | 2,39 | 3,81 | 2,01 |
| | leicht | 13 | 2,76 | 2,18 | 3,59 | 2,33 | 4,51 | 2,56 |
| | erhöht | | | | | | | |
| | erhöht | 9 | 4,53 | 1,93 | 4,74 | 1,81 | 5,24 | 1,53 |
| | Gesamt | 30 | 3,38 | 2,08 | 3,83 | 2,22 | 4,54 | 2,15 |
| Insulin | normal | 8 | 23,84 | 18,91 | 45,78 | 48,77 | 31,36 | 23,73 |
| | leicht | 13 | 27,88 | 25,10 | 44,39 | 46,88 | 38,48 | 38,55 |
| | erhöht | | | | | | | |
| | erhöht | 9 | 28,29 | 16,00 | 46,96 | 25,78 | 35,29 | 28,38 |
| | Gesamt | 30 | 26,92 | 20,53 | 45,53 | 40,84 | 35,62 | 31,33 |
| Proinsulin | normal | 8 | 25,35 | 23,66 | 28,65 | 24,09 | 16,25 | 10,81 |
| | leicht | 13 | 25,18 | 19,53 | 18,16 | 13,89 | 16,57 | 8,53 |
| | erhöht | | | | | | | |
| | erhöht | 9 | 32,82 | 17,80 | 32,20 | 20,32 | 39,52 | 28,85 |
| | Gesamt | 30 | 27,52 | 19,82 | 25,17 | 19,35 | 23,37 | 20,09 |
| HbA1C | normal | 8 | 6,55 | 0,61 | 6,31 | 0,78 | 6,61 | 0,61 |
| | leicht | 13 | 6,91 | 1,13 | 6,89 | 1,12 | 6,95 | 0,91 |
| | erhöht | | | | | | | |
| | erhöht | 9 | 7,19 | 0,69 | 7,11 | 0,75 | 7,28 | 0,82 |
| | Gesamt | 30 | 6,90 | 0,90 | 6,80 | 0,95 | 6,96 | 0,83 |
| Cholesterin | normal | 8 | 6,22 | 1,97 | 6,59 | 1,43 | 6,15 | 1,34 |
| | leicht | 13 | 5,11 | 0,70 | 5,15 | 0,83 | 4,92 | 0, 93 |
| | erhöht | | | | | | | |
| | erhöht | 9 | 5,66 | 1,27 | 5,78 | 1,45 | 5,84 | 1,33 |
| | Gesamt | 30 | 5,57 | 1,34 | 5,72 | 1,31 | 5,52 | 1,26 |
| HDL-Cholesterin | normal | 8 | 1,41 | 0,32 | 1,50 | 0,41 | 1,48 | 0,32 |
| | leicht | 13 | 1,27 | 0,27 | 1,26 | 0,25 | 1,25 | 0,29 |
| | erhöht | | | | | | | |
| | erhöht | 9 | 1,20 | 0,23 | 1,29 | 0,20 | 1,23 | 0,26 |
| | Gesamt | 30 | 1,28 | 0,28 | 1,33 | 0,30 | 1,30 | 0,30 |
| LDL-Cholesterin | normal | 8 | 4,15 | 1,49 | 4,24 | 1,05 | 3,83 | 1,08 |
| | leicht | 11 | 3,09 | 0,45 | 3,11 | 0,70 | 2,86 | 0,72 |
| | erhöht | | | | | | | |
| | erhöht | 8 | 3,48 | 1,07 | 3,40 | 1,23 | 3,31 | 1,02 |
| | Gesamt | 27 | 3,52 | 1,09 | 3,50 | 1,04 | 3,26 | 0,97 |
| Triglyceride | normal | 8 | 1,47 | 0,99 | 1,83 | 0,99 | 1,82 | 1,26 |
| | leicht | 13 | 2,05 | 1,77 | 1,73 | 0,91 | 2,12 | 1,54 |
| | erhöht | | | | | | | |
| | erhöht | 9 | 2,39 | 1,51 | 2,85 | 1,66 | 3,41 | 2,26 |
| | Gesamt | 30 | 1,99 | 1,52 | 2,09 | 1,26 | 2,42 | 1,80 |
| Blutzucker | normal | 8 | 8,92 | 4,45 | 8,62 | 4,06 | 8,85 | 2,61 |
| | leicht | 13 | 8,69 | 4,11 | 9,67 | 4,15 | 10,77 | 4,78 |
| | erhöht | | | | | | | |
| | erhöht | 9 | 9,71 | 4,56 | 9,65 | 2,18 | 9,89 | 2,76 |
| | Gesamt | 30 | 9,06 | 4,21 | 9,38 | 3,55 | 9,99 | 3,72 |

### Anwendungsbeispiel 2

### Klinische Erfahrungen mit Diabetes mellitus Typ 2 Patienten bei der Kombination von Höhentraining (normobarer Hypoxie) mit der Gabe von aktiviertem Zeolith kombiniert mit Extrakten von Pflanzen der Gattung Urticaceae und Omega-3-Fettsäuren

8 Patienten wurden 3 Wochen lang mit einem 30-minütigen Laufbandtraining in einer simulierten Höhe von 1500mbelastet. Zusätzlich erhielten diese Patienten pro Tag 1000mg Omega-3-Fettsäuren und siliziumhaltige Mineralstoffe (aktivierter Klinopthilolith/Brennesselextrakt, 3x2 Kapseln, entspricht 6 Gramm). Laborchemisch wurden davor und danach folgende Parameter bestimmt: C-Peptid, Insulin, Proinsulin, HBA₁C, Cholesterin, HDL-Cholesterin, LDL-Cholesterin, Triglyceride, Blutzucker, Insulinbedarf, Körpergewicht.

Dabei wurden folgende Veränderungen beobachtet: C-Peptid-unverändert, Insulin unverändert, Proinsulin-leicht vermindert, HBA₁C- leicht vermindert, Cholesterin-deutlich vermindert, HDL-Cholesterin - unverändert, LDL-Cholesterin - deutlich vermindert, Triglyceride-leicht vermindert, Blutzucker- deutlich vermindert, Insulinbedarf - sehr deutlich vermindert, Körpergewicht - sehr deutlich vermindert.

Aus den vorliegenden Ergebnissen ist eine klinische Relevanz im Sinne einer positiven Beeinflussung des metabolischen Syndroms zu erkennen.

### Abbildungsbeschreibung:

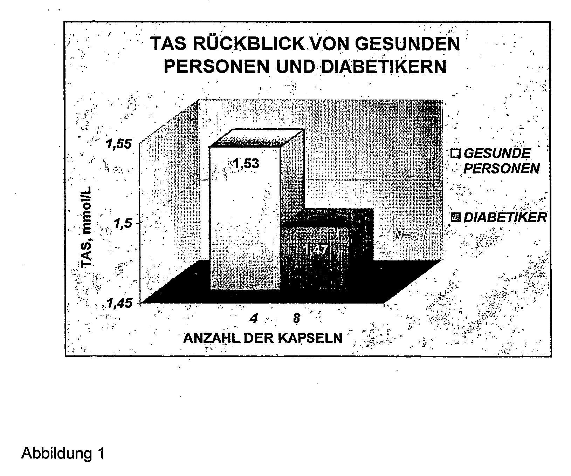

Abbildung 1 zeigt die Werte des Totalen Antioxydanten Status (TAS) von Diabetikern, die vor der Einnahme von aktiviertem Klinoptilolith mit Brennesselextrakt einen TAS von 1,28 mmol/L hatten und nach einmonatiger Einnahme von durchschnittlich 4-8 Kapseln aktiviertem Klinoptilolith mit Brennesselextrakt pro Tag (2-4 Gramm Gesamtdosis) einen TAS-Wert von 1,53-1,47 mmol/l entwickelten.

## Patentansprüche

1. Mittel für die Verwendung zur Therapie und Prophylaxe von Diabetes mellitus Typ 1 und Typ 2, umfassend physikalisch und/oder chemisch aktivierte natürliche oder synthetische Zeolithe, Extrakte von Pflanzen der Gattung Urticaceae, Omega-3-Fettsäuren und gegebenenfalls weitere Stoffe unter den Bedingungen normobarer Hypoxie.

2. Mittel nach Anspruch 1, wobei als natürliche Zeolithe Heulandit/Klinoptilolith, Natrolith oder Thomsonit verwendet werden.

3. Mittel nach Anspruch 2, **dadurch gekennzeichnet, dass** mikronisiertes Heulandit/Klinöptilolith mit einem Teilchendurchmesser von weniger als 5 µm verwendet wird.

4. Mittel nach Anspruch 1-3, wobei als synthetische Zeolithe NaY Zeolith verwendet wird.

5. Mittel nach Anspruch 1-4, das die folgenden Bestandteile in folgenden Mengen umfasst:
- Physikalisch und/oder chemisch aktivierte Mineralstoffe 70-98%
- Extrakte von Pflanzen der Gattung Urticaceae 2-30%.

6. Mittel nach Anspruch 5, das die folgenden Bestandteile in folgenden Menger enthält:
| | |
|---|---|
| Physikalisch und/oder chemisch aktivierte Mineralstoffe | 75% |
| Extrakte von Pflanzen der Gattung Urticaceae | 25%. |

## Claims

1. Means for the usage in therapy and prophylaxis of Type 1 and Type 2 Diabetes mellitus, entailing physically and/or chemically activated natural or synthetic zeolites, extracts of plants of the Urticaceae species, Omega-3 fatty acids and, if applicable, further substances of conditions of normobar hypoxia.

2. Means according to Claim 1, in which heulandite/klinoptilolith, natrolith or thomsonite are used as natural zeoliths.

3. Means according to Claim 2, wherein micronised heulandite/klinoptilolith with a particle diameter of less than 5 µm is used.

4. Means according to Claim 1-3, in which NaY zeolith is used as a synthetic zeoliths.

5. Means according to Claim 1-4, containing the following components in the following quantities:
- Physically and/or chemically activated minerals 70-98%
- Extracts of plants of the Urticaceae species 2-30%.

6. Means according to Claim 5, containing the following components in the following quantities:
| | |
|---|---|
| Physically and/or chemically activated minerals | 75% |
| Extracts of plants of the Urticaceae species | 25%. |

## Revendications

1. Produit pour un emploi dans l'objectif du traitement et de la prophylaxie du diabète mellitus (diabète sucré) de type 1 et type 2, comprenant des zéolithes naturelles ou synthétiques, activées par procédé physique et/ou chimique, des extraits de plantes de la famille des urticacées, des acides gras oméga 3 et le cas échéant d'autres substances dans les conditions de l'hypoxie normobare.

2. Produit selon la revendication 1, en utilisant de l'heulandite/la clinoptilolite, de la natrolite ou de la thomsonite en tant que zéolithe.

3. Produit selon la revendication 2, **se caractérisant par le fait que** l'heulandite/clinoptilolite utilisée est micronisée et a un diamètre de particules inférieur à 5 µm.

4. Produit selon la revendication 1-3, en utilisant de la zéolithe NaY en tant que zéolithe synthétique.

5. Produit selon la revendication 1-4 qui comprend les éléments suivants dans les quantités suivantes :
- minéraux activés par procédé physique et/ou chimique 70-98 %
- extraits de plantes de la famille des urticacées 2-30 %.

6. Produit selon la revendication 5 qui comprend les éléments suivants dans les quantités suivantes :
| | |
|---|---|
| minéraux activés par procédé physique et/ou chimique | 75 % |
| extraits de plantes de la famille des urticacées | 25 %. |
